# EUROPÄISCHE PATENTSCHRIFT

(11) **EP 0 592 726 B1**
(45) Veröffentlichungstag und Bekanntmachung des Hinweises auf die Patenterteilung: **05.03.1997**
(21) Anmeldenummer: 92203134.9
(22) Anmeldetag: 12.10.1992
(51) Int. Cl.: A61F 2/06

(54) **Katheter mit einer Gefässstütze**
Catheter with a vessel support
Cathéter avec un support vasculaire

(43) Veröffentlichungstag der Anmeldung: 20.04.1994
(73) Patentinhaber: SCHNEIDER (EUROPE) AG, CH-8180 Bülach (CH)
(72) Erfinder: Gianotti, Marc, CH-8542 Wiesendangen (CH); Lehmann, Paul, CH-8404 Winterthur (CH)
(74) Vertreter: Misrachi, Alfred

(56) Entgegenhaltungen:
- EP-A- 0 321 912
- EP-A- 0 408 245
- EP-A- 0 418 677
- EP-A- 0 433 447
- DE-B- 1 250 058
- US-A- 4 665 918
- US-A- 5 071 407

## Beschreibung

Die Erfindung betrifft einen Katheter mit einer zylindrischen Gefässstütze aus einem durchlässigen Netz sich überkreuzender steifer Fäden, wobei die Gefässstütze bei ihrem Einsatz sich aus einem gespannten Zustand mit kleinem Umfang durch ihre radiale Elastizität aus eigener Kraft aufweitet in einen die Gefässwand stützenden entspannten Zustand mit über die Länge gleichbleibendem grossem Umfang, mit einem schlauchförmigen äusseren Katheterschaft, der an seinem distalen Ende die gespannte Gefässstütze in sich aufnimmt und aus dem die Gefässstütze zu ihrem Einsatz freigegeben werden kann, mit einem Innenkatheter im Innern des schlauchförmigen äusseren Katheterschafts, der im Aussenkatheter verschieblich ist, der für eine direkte Übertragung der Verschiebewege entlang deiner Länge im Aussenkatheter gelagert ist und der in seinem Inneren eine Passage für einen Führungsdraht freilässt, die er eng umschliesst, wobei zur Freigabe der Gefässstütze der äussere Katheterschaft gegenüber dem Innenkatheter zurückgezogen wird und wobei die Gefässstütze an ihrem proximalen Ende im eingespannten Zustand so gesichert ist, dass sie einen mit der Gefässstütze sich selbst öffnenden durchlässigen Netzkegel bildet, dessen Radius stetig zum Radius der entspannten Gefässstütze ansteigt und wobei die Gefässstütze mit Hilfe des Netzkegels fest an dem lediglich Druck- und Zugkräfte übertragenden Innenkatheter verankert ist.

Solche Katheter weisen an ihrem distalen Ende eine Gefässstütze auf, die nur vorübergehend in den Körper eingesetzt wird, um eine Gefässwand während einer gewissen Zeitspanne abzustützen. Danach soll die Gefässstütze wieder aus dem Gefäss entfernt werden.

Unbefristet im Körper verbleibende Stützen für die Gefässwand werden beispielsweise nach einer Ballondilatation eines Herzkranzgefässes eingesetzt. Eine Hauptkomplikation bei dieser Technik besteht nämlich darin, dass durch die gewaltsame Aufweitung des Blutgefässes Teile der Intima, der innersten Schicht der Gefässwand, sich von der Gefässwand loslösen können und dann mehr oder weniger stark den Durchfluss im Gefäss behindern können. Im schlimmsten Fall kann ein losgelöstes Stück Gefässwand wie eine Ventilklappe wirken, die den Durchfluss ganz versperrt. An bestimmten Behandlungsorten, z. B. eben gerade in den Koronararterien, führt das zu einer kritischen Situation, die eine notfallmässige Bypass-Operation mit hohem Risiko für den Patienten notwendig macht. Aber auch an anderen Behandlungsorten und bei weniger ungünstigem Verlauf der Komplikation ist auf jeden Fall durch diesen Effekt der angestrebte Erfolg der Behandlung verhindert.

Im Falle solcher Komplikationen werden deshalb seit einiger Zeit die beispielsweise aus der US 4 655 771 bekannten Gefässstützen an der behandelten Stelle in das Gefäss eingesetzt um das Gefäss von innen offen zu halten.

Dazu wird durch denselben Einstich, der schon für den Ballonkatheter benutzt worden ist, ein Katheter in das Blutgefäss eingeführt. In diesem Katheter liegt innen an seinem vom Bediener entfernten, distalen Ende die Gefässstütze. Die Gefässstütze ist zylindrisch und besteht aus einem Netz sich überkreuzender steifer Fäden. Sie ist vom selbstexpandierenden Typ, d.h. sie ist in gespanntem Zustand in den Katheter eingelegt und entspannt sich ohne Hilfe aus eigener Kraft. Andere Gefässstützentypen müssen z.B. durch einen innenliegenden Ballon in ihren aufgeweiteten Zustand gebracht werden. Am Behandlungsort wird die Gefässstütze durch Zurückziehen des Aussenkatheters aus dem Katheter freigegeben und vom Katheter gelöst. Ein im Katheter liegender verschieblicher Innenkatheter dient der Gefässstütze als Widerlager solange der Aussenkatheter zurückgezogen wird. Die Gefässstütze verbleibt nach ihrer Freigabe im Gefäss, sie stützt das Gefäss permanent. Der Katheter dagegen wird wie üblich zurückgezogen und die Einstichstelle in das Gefäss wird verschlossen.

Solche Gefässstützen erfüllen soweit ihren Zweck, als sie die losgelöste innerste Gefässschicht, die Intima, wieder an die Gefässwand drücken und damit das Gefäss für den Durchfluss offen halten. Schwierigkeiten treten aber insofern auf, als diese Gefässstützen, da sie ja Fremdkörper in dem Gefäss darstellen, Blutgerinnsel verursachen können. Dieser Gefahr muss mit hohen, potentiell gefährlichen Dosen von gerinnungshemmenden Medikamenten begegnet werden. Nach einigen Wochen wird die Gefässstütze dann von der Gefäss-Innenhaut, dem Endothel überwachsen und die Gefahr von Blutgerinnseln ist dadurch weitgehend gebannt. Jetzt tritt eine neue Schwierigkeit auf. Es zeigt sich nämlich, dass die Gewebezellen, die durch das Einbringen der Gefässstütze in ihrem Wachstum angeregt wurden, in einigen Fällen nicht mehr aufhören zu wachsen. Es kann daher zu einem erneuten teilweisen oder vollständigen Verschluss des Gefässes kommen.

Es ist aber inzwischen bekannt, dass eine losgelöste Intima schon in relativ kurzer Zeit wieder mit der Gefässwand verbunden und ausgeheilt werden kann. Teilweise reicht dazu ein erneutes kurzzeitiges Auffüllen des Ballons am Ende des beschriebenen Ballonkatheters. Während der Ballon gespannt ist, ist aber in dem entsprechenden Gefäss der Blutfluss unterbrochen. Diese Methode ist deshalb nicht an allen Behandlungsorten einsetzbar. Ausserdem werden die Ausheilzeiten durch eventuell während der Behandlung notwendige gerinnungshemmende Medikamente verlängert. Entsprechend müsste man die Unterbrechung der Blutzufuhr verlängern wenn man in diesem Fall die Intima mit Hilfe eines Ballons an die Gefässwand pressen wollte.

Um den geschilderten Schwierigkeiten aus dem Wege zu gehen, sind deshalb schon Katheter vorgeschlagen worden, bei denen die Gefässstütze wieder aus dem Gefäss entfernt werden kann. Es verbleibt in diesem Fall kein Implantat im Körper und die damit verbundenen Komplikationen bleiben aus. Sobald die Gefässstütze ihre Aufgabe erfüllt hat und die Intima wieder mit der Gefässwand verbunden ist, kann dieses Hilfsmittel wieder aus dem Körper abgezogen werden.

Ein Beispiel dafür ist in der ER 0 321 912 A1 dargestellt. Es handelt sich dabei um eine Gefässstütze aus einem in die Länge gestreckten Netzschlauch aus verwobenen Drähten, der in gestreckter Form in das Gefäss eingeführt wird. Am Behandlungsort werden die beiden Enden des Netzschlauches aufeinander zu bewegt. Das Netz zwischen den Enden beult sich dadurch aus zu einer Hohlform, die sich an die Gefässinnenwand legt und sie stützt. Das Netz, aus dem die Gefässstütze besteht, ist also in diesem Fall nicht selbstexpandierend, sondern es hat seine entspannte Lage im gestreckten Zustand. In diesem entspannten gestreckten Zustand mit kleinem Umfang wird die Gefässstütze in das Gefäss eingeführt und nach Gebrauch auch wieder aus dem Gefäss entfernt. Je nachdem mit welcher Kraft die Enden des Netzschlauches aufeinander zu bewegt werden, entsprechend stark ist der Druck der Hohlform auf die Gefässwand. Nachteilig an dieser Ausführung ist allerdings, dass die einzelnen Drähte knicken können, wenn die Enden des Netzschlauches zu stark gegeneinander gezogen werden, und die Drähte im Gefäss nicht ausweichen können. Mit geknickten Drähten ist der Katheter nur unter Komplikationen aus dem Gefäss zu entfernen, weil die Betätigungselemente des Netzes nur bedingt Druckkräfte übertragen können um das Netz wieder in die gestreckte Form mit kleinenm Umfang zurück zu bringen. Nachteilig ist ausserdem, dass die Betätigungskräfte zum Offenhalten des Netzschlauches während der Behandlungsdauer über eine relativ weite Distanz hinweg von aussen aufrecht erhalten werden müssen. Es können sich dabei Übertragungsfehler einstellen wenn zum Beispiel der Katheter zwischen dem Einstich in die Haut und dem Behandlungsort bewegt wird.

Ein weiteres Beispiel für eine wieder aus dem Körper entfernbare Gefässstütze ist in der WO 91/07 928 gezeigt. In diesem Fall wird die Gefässstütze aus einem schraubenförmig gewundenen einzigen Draht gebildet. Der Draht ist gestreckt in einem dünnen Katheterrohr aufgenommen und wird daraus hervorgeschoben. Sobald der Draht vorne aus dem dünnen Katheterrohr austritt, nimmt er wegen der in ihm vorgesehenen Spannungen eine Spiral- oder Schraubenform an. Die einzelnen Windungen der Schraubenform drängen radial nach aussen und stützen die Gefässwand. Zum Entfernen der Gefässstütze wird der Draht wieder in den Katheter zurückgezogen. Der Draht nimmt dabei wieder seine gestreckte Form ein. Diese Gefässstütze ist damit vom selbstexpandierenden Typ. Beim selbstexpandierenden Typ droht nicht die Gefahr, dass übermässige Bedienungskräfte die Gefässstütze beeinträchtigen oder unbrauchbar machen. Die Verwendung nur eines Drahtes bringt allerdings den Nachteil mit sich, dass die einzelnen Windungen der Gefässstütze sehr eng nebeneinander angeordnet werden müssen um die Gefässwand flächenmässig und damit wirkungsvoll abzustützen. In einer Schraubenform mit nur einem Draht haben ausserdem die Windungen untereinander keinen Zusammenhalt, der einen gleichmässig engen Abstand der Windungen sicherstellen würde. Es können somit Lücken in der Abstützung der Gefässwand entstehen. Ein unangenehmer Nachteil liegt auch darin, dass die schraubenförmige Gefässstütze beim Einsetzen und beim Entfernen aus dem Gefäss nicht stationär bleibt. Beim Einsetzen und beim Entfernen der Gefässstütze muss der jeweils entspannte Teil der Schraubenfeder sich gegenüber dem Draht im Katheter drehen um den unterschiedlichen Zustand des Drahtes auszugleichen. Beim Setzen der Gefässstütze kann dabei das freie sich drehende Ende des Drahtes in der Gefässwand Verletzungen hervorrufen. Vor allem aber kann beim Setzen die schraubenförmige Gefässstütze durch ihre Drehung unter einen losgelösten Lappen der Gefässwand wandern und sich damit die Erfüllung ihrer Aufgabe vereiteln. Diese Gefässstütze ist daher nicht so sicher wirksam wie z.B. die bekannte permanent im Gefäss verbleibende Gefässstütze. Schwierigkeiten können auch daraus resultieren, dass am proximalen Ende des Katheters die gesamte Ausstosskraft für die Gefässstütze von nur einem Draht übertragen werden muss.

Ein weiteres Beispiel für eine wieder aus dem Gefäss entfernbare Gefässstütze ist in der EP 0 423 916 A1 veröffentlicht. Es handelt sich um ein in Form eines Schlauchmantelstücks zusammengesetztes Scherengitter aus einem nichtrostenden Stahldraht. Diese Gefässstütze ist ebenfalls vom selbstexpandierenden Typ und wird genau wie die Gefässstütze nach der US 4 655 771 durch Zurückziehen eines Aussenkatheters gegenüber einem Innenkatheter in das Gefäss eingesetzt. Am bedienungsnahen, proximalen Rand des zu einem Schlauchmantelstück zusammengesetzten Scherengitters ist am Rand des Schlauchmantels ein Faden angebracht. Mit diesem Faden kann der Schlauchmantel an seinem proximalen Ende zusammengeschnürt werden. Um dies zu erreichen, werden beide Enden dieses Fadens bis ausserhalb des Körpers geführt und dort lose gesichert solange die Gefässstütze im Gefäss ist. Soll die Gefässstütze entfernt werden, wird über diese beiden Fäden ein neuer Katheter bis zur Gefässstütze vorgeschoben und durch Zug an den Fäden wird das proximale Ende der Stütze zusammengeschnürt. Sodann wird ein zweiter entsprechend grösserer Katheter über den ersten geschoben. Die Gefässstütze wird jetzt mit den Fäden so weit zusammengezogen, bis sie in den grösseren Katheter passt und in ihn hineingezogen werden kann. Danach werden beide Katheter zusammen mit der Gefässstütze entfernt. Nachteilig an dieser Anordnung ist der grosse Aufwand der für diese Anordnung getrieben werden muss. Die Bedienung ist sehr umständlich durch die Handhabung der Fäden und das Setzen mindestens eines neuen Katheters, der z. B. auch über die Fäden geschoben werden muss. Der instrumentelle Aufwand ist ebenfalls sehr hoch, es muss mindestens ein zusätzlicher Katheter vorgesehen werden, der die Gefässstütze wieder aufnimmt. Der zum Setzen der Gefässstütze benutzte Katheter ist dazu zu klein. Beim Einsatz der Gefässstütze in den Koronararterien ist beim Einziehen der Gefässstütze in den grösseren Katheter ausserdem mit Schwierigkeiten zu rechnen, weil die Koronararterien ständig in Bewegung sind. Die Schwierigkeiten kommen dann daher, dass die am proximalen Ende zusammengezogene Gefässstütze nicht immer genau mittig zum grösseren Katheter liegt und die Gefässstütze sich auch nicht selbst gegenüber dem grösseren Katheter zentriert. Die Gefässstütze bleibt am Rand des grösseren Katheters hängen.

Der den Oberbegriff bildende Katheter mit Gefässstütze ist vor dem Anmeldetag dieser Anmeldung und nach dem Anmeldetag der europäischen Anmelding 92 200 294.4 EP-A-554 579 bekannt geworden und entspricht der dortigen Beschreibung.

Bei diesem Katheter wird eine selbstexpandierende Gefässstütze nach der US 4 655 771 aus einem durchlässigen Netz sich überkreuzender Fäden an den proximalen Enden der Netzfäden im zusammengefalteten, eingespannten Zustand an einem Innenkatheter verankert. Während der Katheter im Gefäss vorgeschoben wird, ist die Gefässstütze in einem entsprechenden Aussenkatheter eingespannt gelagert. Sobald der Aussenkatheter gegenüber dem Innenkatheter zurückgezogen wird, tritt die Gefässstütze distal aus dem Aussenkatheter heraus und entspannt sich. Durch die Verankerung am Innenkatheter in eingefaltetem Zustand bildet sich im entspannten Zustand der Gefässstütze an ihrem proximalen Ende ein Netzkegel. Die Kegelform des Netzes und die Verankerung am Innenkatheter ermöglicht, dass die Gefässstütze nach Gebrauch durch Vorschieben des Aussenkatheters zuverlässig wieder in den Aussenkatheter zurückgefaltet werden kann und damit auch wieder aus dem Gefäss entfernt werden kann.

Dieser Katheter entspricht weitgehend den an ihn gestellten Anforderungen, unbefriedigend an diesem Katheter ist allerdings noch die Phase im Bedienungsablauf wenn die gespannte Gefässstütze innerhalb des Aussenkatheters liegt und der Katheter im Gefäss vorgeschoben wird. In dieser Phase muss der Aussenkatheter durch eine Schutzkappe verschlossen werden. Anderenfalls würde sich der Aussenkatheter beim Vorschieben im Gefäss mit der jeweiligen Körperflüssigkeit aufladen. Vor allem wäre dann die Gefässwand direkt dem sich vorschiebenden Rand der distalen Aussenkatheteröffnung ausgesetzt. Dadurch können Verletzungen verursacht werden. Zur Vermeidung generell von Verletzungen während des Vorschiebens eines Katheters in einem Gefäss wird normalerweise ein Führungsdraht eingesetzt, der den Katheter von innen führt und über den der Katheter vorgeschoben wird. Bei dem hier vorliegenden Katheter kann der Einsatz eines Führungsdrahtes die Verletzungsgefahr nicht nachhaltig beseitigen. Ein Führungsdraht muss einerseits so dünn und flexibel wie möglich sein, auf jeden Fall müssen mit Sicherheit Verletzungen durch den Führungsdraht selbst ausgeschlossen werden. Die distale Öffnung des Aussenkatheters durch die der Führungsdraht läuft, muss andererseits um ein vielfaches grösser sein als der Führungsdrahtdurchmesser, da sie ja die Gefässstütze aufnehmen muss. Wegen des grossen Durchmessersprunges zwischen dem Führungselement einerseits und dem zu Führenden Element andererseits kann ein Führungsdraht diesen Katheter nicht mit der notwendigen Sicherheit führen und dadurch etwa Verletzungen vermeiden. Die Schutzkappe die am distalen Ende des Katheters vorgesehen ist, bietet ebenfalls Probleme. Einerseits soll sie in geschlossenem Zustand die distale Aussenkatheteröffnung sicher verschliessen und die Gefässwand sicher vor Verletzungen schützen. Andererseits soll sie aber dem Austreten und dem Einfalten der Gefässstütze möglichst keinen Widerstand entgegensetzen. Es ist schwierig, beide Forderungen gleichzeitig zu erfüllen. Vor allem beim Zurückfalten der Gefässstütze in den Aussenkatheter ist bei Gefässstützen, die aus einem grossmaschigen Netz hergestellt sind, mit Schwierigkeiten zu rechnen. Neben diesen geschilderten Aufgaben kann die Schutzkappe nicht noch ausserdem den Katheter am Führungsdraht entlang führen. Das würde der Forderung widersprechen, dass sie auch ein leichtes Austreten der Gefässstütze zulassen soll. Ein Führungsdraht liegt bei diesem Katheter deshalb praktisch ohne spürbare Führung unkontrolliert in der um ein vielfaches grösseren distalen Öffnung des Aussenkatheters.

Die Erfindung hat sich daher die Aufgabe gestellt, einen Katheter mit einer nach einer vorrübergehenden Einsatzzeit zuverlässig wieder aus dem Gefäss entfernbaren Gefässstütze bereitzustellen, der sich sicher geführt entlang einem Führungsdraht vorschieben lässt, der beim Vorschieben im Gefäss keine Gefässflüssigkeit aufsammelt und der Verletzungen der Gefässwand durch den Rand der distalen Katheteröffnung verhindert.

Diese Aufgabe wird dadurch gelöst, dass die das Netz bildenden steifen Fäden an ihren Enden wieder zusammengeführt werden und distal an einer Spitze verankert sind und dadurch am distalen Ende der Gefässstütze einen zweiten, zum ersten spiegelbildlich liegenden Netzkegel bilden, wobei die Spitze, an der die Fäden verankert sind, in ihrem Inneren den Führungsdraht durchlässt und nach aussen so geformt ist, dass sie die distale Öffnung des Aussenkatheters verschliesst wenn der Aussenkatheter die Gefässstütze gespannt vollständig in sich aufnimmt, wobei die Spitze, wenn sie den Aussenkatheter verschliesst, mit ihrem distalen Ende gegenüber der distalen Öffnung des Aussenkatheters sich verjüngend soweit nach distal erstreckt, dass der volle Aussendurchmesser des Katheters nicht stumpfwinklig auf die Gefässwand auftreffen kann.

Die Spitze, an der die distalen Enden der Fäden verankert sind, übernimmt damit die Funktionen der Schutzkappe des bekannten Katheters. Sie stellt einen Verschluss dar, der verhindert, dass der Aussenkatheter beim Vorschieben im Gefäss sich mit Körperflüssigkeit aufläd oder vielleicht Ablagerungen von den Gefässwänden abstreift und sie aufsammelt. Der Verschluss ist in diesem Fall aber nicht wie im Stand der Technik am Aussenkatheter befestigt, sondern an der Gefässstütze und zwar an deren distalem Ende. Sie bewegt sich dadurch zusammen mit der Gefässtütze und sie verschliesst damit selbttätig die distale Aussenkatheteröffnung sobald die Gefässstütze gegenüber dem Aussenkatheter zurückgezogen wird. Anders als die Schutzkappe steht die Spitze der freien Entfaltung und Zurückfaltung der Gefässstütze nicht im Wege, es entsteht bei dieser Anordnung kein Widerstreit mehr zwischen den Forderungen nach einerseits sicherem Verschluss mit gutem Verletzungsschutz und nach andererseits sicherer Freigabe und zuverlässiger Zusammenfaltung der Gefässstütze. Die Spitze nimmt gleichzeitig auch die Führungskräfte vom Führungsdraht auf, da sie den Führungsdraht in sich aufnimmt. Sie überträgt die Führungskräfte auf den Katheter und gewährleistet dadurch eine einwandfreie sichere Führung des Katheters entlang dem Führungsdraht. Der Katheter kann blindlings, schnell und sicher und ohne Verletzungsgefahr entlang dem Führungsdraht vorgeschoben werden. Dadurch dass die Spitze sich verjüngend soweit nach distal erstreckt gegenüber der Öffnung des Aussenkatheters dass der volle Aussendurchmesser des Katheters nicht stumpfwinklig auf die Gefässwand auftreffen kann, verhindert die Spitze mögliche Verletzungen der Gefässwand durch den Katheter beim Vorschieben. Die Gefässwand wird nicht mehr ungeschützt dem relativ dünnen und damit potentiell traumatischen, verletzenden Aussenkatheterrand ausgesetzt. Schliesslich wird durch die Spitze auch noch eine Verletzungsgefahr durch die distalen Enden der Gefässstützenfäden beseitigt. Beim Vorschieben der bekannten distal offenen Gefässtütze im Gefäss spreizen sich nämlich die distalen Enden der Gefässstützenfäden ausseinander und bohren sich auswärts gerichtet in die Gefässwand. Es ist zwar unwahrscheinlich, dass die Gefässstütze während der Bedienung versehentlich vorgeschoben wird, das wäre eine grobe Fehlbedienung des Katheters auch weil die Gefässstütze dadurch gestaucht wird und die Gefässstützenfäden knicken können. Während der Zeit, in der die Gefässstütze gebraucht wird, liegt aber auch die gesamte restliche Länge des Katheters noch im Körper des Patienten. Das Verletzungsrisiko resultiert dann weniger aus möglichen Fehlbedienungen des Katheters sondern aus möglichen, vielleicht unvermeidbaren Patientenbewegungen. Die Bewegungen des Patienten können sich auf den Innenkatheter übertragen und können dadurch auch einen Druck auf die Gefässtütze ausüben. Die in der Erfindung vorgesehene Spitze, die alle distalen Enden der Gefässstützenfäden auf sich vereinigt und sie sichert, schliesst jetzt auch für diesen Fall nachhaltig jede Verletzungsgefahr für den Patienten aus.

Wenn die Spitze eine Schulter aufweist, die den Aussenkatheter übergreift wird eine besonders gute Abdichtung der distalen Katheteröffnung errreicht. Beim Vorschieben des Aussenkatheters ergibt sich ein deutlich spürbarer Anschlag wenn die Gefässstütze wieder vollständig eingefaltet ist. Die Spitze kann in diesem Fall auch gleichzeitig als Dilator dienen bei engen Gefässpassagen und beim Einführen des Instrumentes an der Punktionsstelle. Die Kosten eines speziellen Einführbesteckes und der Zeitaufwand für dessen Handhabung werden gespart. In Notfallsituationen ist das ein Gesichtspunkt von besonderem Interesse.

Wenn die Spitze röntgenologisch erkennbar ist, kann auch die Lage des distalen Endes der Gefässstütze durch Röntgenbilder kontrolliert werden, ohne dass für die Gefässstützenfäden speziell radiopakes Material benutzt werden müsste. Der Arzt hat dann selbst bei bereits plazierter Gefässstütze eine genaue Kontrolle über die Lage auch des distalen Endes der Stütze. Er kann dann eventuelle Fehlplazierungen oder Verschiebungen genau beurteilen und gegebenenfalls korrigieren.

Eine besonders vorteilhafte Anordnung ergibt sich, wenn an der Spitze ein radiopaker Ring angebracht ist, auf dem die distalen Enden der Gefässstützenfäden verankert sind. Diese Massnahmen ergeben durch die Zusammenfassung von zwei Funktionen an einem Ort eine einfache Spitzenform und ein leicht und einfach zu montierendes Instrument. Der radiopake Ring stört so angeordnet nicht die aussen liegende Oberfläche, die mit der Gefässwand in Berührung kommt, er behindert auch keine andere Funktion der Spitze.

Ein besonders vorteilhaftes Verfahren zur Herstellung eines erfindungsgemässen Katheters ergibt sich, wenn die konvergierenden Enden der beiden Netzkegel der Gefässstütze jeweils durch einen Schrumpfschlauch auf dem Aussendurchmesser des entsprechenden Verankerungsbettes am Innenkatheter bzw. auf der Spitze zusammengehalten werden während die Netzkegel mit dem Innenkatheter bzw. mit der Spitze verbunden werden. Dadurch wird durch eine einfache Massnahme das Netz der Gefässstütze von allen Seiten gleichmässig eingespannt und die Verbindung durch Wärmebehandlung oder mit Hilfe von Bindemitteln kann ungestört hergestellt werden. Ein besonderer Vorteil in dieser Methode liegt auch darin, dass die Einspannung eine konstante Kraft ausübt, auch wenn der eingespannte Gegenstand nachgibt oder gar im Durchmesser kleiner wird. Diese Eigenart des vorgeschlagenen Verfahrens ist wichtig, wenn die Verbindung durch Wärmebehandlung hergestellt wird, aber auch wenn bei Verbindung mit Bindemitteln der kleinstmögliche Durchmesser der fertigen Verbindung erzielt werden soll. Durch dieses Verfahren kann daher ein Katheter mit besonders kleinem Aussendurchmesser hergestellt werden.

Nachfolgend wird die Erfindung anhand von Ausführungsbeispielen in den Zeichnungen näher erläutert. Es zeigen:
- Fig. 1: eine Ansicht eines Katheters nach der Erfindung mit freigesetzter Gefässstütze in einem schematisch angegebenen Gefäss
- Fig. 2: einen Längsschnitt durch einen Katheter nach der Erfindung mit eingespannter Gefässstütze
- Fig. 3: ein Detail eines Längsschnitts durch ein anderes Ausführungsbeispiel eines Katheters nach der Erfindung mit eingespannter Gefässstütze.

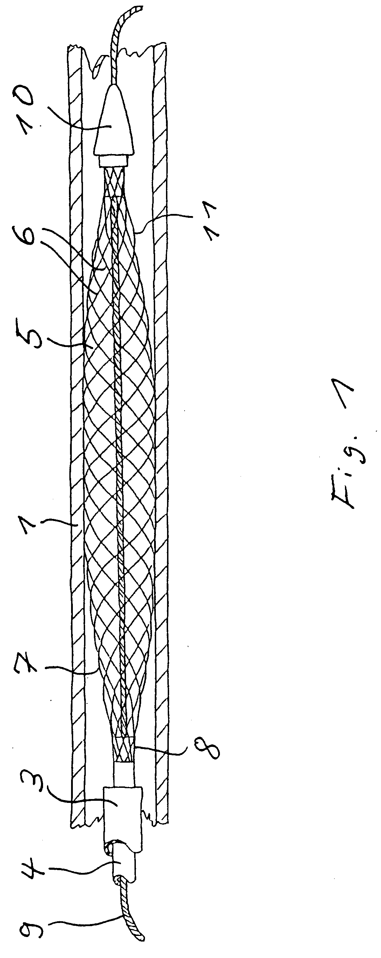

In Fig. 1 ist mit 1 idealisiert ein Gefäss z.B. eines menschlichen Körpers dargestellt. Das Gefäss kann Flüssigkeiten, z.B. Blut aufnehmen, es kann aber auch Luft führen, sodass das Gefäss auch eine Luftröhre darstellen kann. In diesem Gefäss liegend ist das bedienungsferne, distale Ende eines Katheters 2 dargestellt. Der Katheter 2 ist an geeigneter Stelle, z.B. an einer Punktion, in das Gefäss eingeführt worden und ist von ausserhalb des Körpers bis zu der dargestellten Stelle im Gefäss vorgeschoben worden. Der Katheter 2 besteht aus einem schlauchförmigen äusseren Katheterschaft 3 und aus einem in diesem liegenden verschieblichen Innenkatheter 4. Der Innenkatheter 4 ist entlang seiner Länge gerade so stramm im Aussenkatheter 3 gelagert, dass eine Längsbewegung des Innenkatheters im Aussenkatheter keine Kräfte erfordert, die keine gefühlvolle Bedienung mehr erlauben. Die Verschiebewege an der proximalen Bedienerseite werden dadurch ohne Hysteresis-Erscheinungen auf das distale Arbeitsende des Innenkatheters 4 übertragen. In der in Fig. 1 dargestellten Lage ist der Innenkatheter 4 aus dem distalen Ende des äusseren Katheterschaftes 3 ein Stück herausgeschoben.

Innen umschliesst der Innenkatheter 4 eng eine Passage für einen Führungsdraht 9.

Ein Führungsdraht ist ein Draht, der so flexibel ausgebildet ist, dass Verletzungen des Gefässes durch den Draht beim Vorschieben des Drahtes ausgeschlossen sind. Ein Führungsdraht kommt auf jeden Fall an der Punktionsstelle zum Einsatz, wo das Gefäss punktiert wird. Dort wird nach der "Seldinger-Technik" nach der Punktion mit einer Hohlnadel zunächst durch die Hohlnadel ein Führungsdraht in das Gefäss eingeführt. Über den Führungsdraht wird dann beispielsweise ein Dilator eines Einführ-Besteckes vorgeschoben. Der Dilator weitet die Punktionsstelle auf, zum Beispiel für einen "Einführungsschleuse" genannten Hohlkatheter, der unmittelbar über den Dilator geschoben wird. Wenn der Dilator wieder entfernt ist, können durch die Einführungsschleuse dann die verschiedensten Katheter in das Gefäss eingebracht werden, vor allem auch solche ohne Dilator an ihrem distalen Ende und solche ohne zentrale Passage, ohne zentrales Lumen für einen Führungsdraht.

Der Innenkatheter 4 umschliesst die Passage für den Führungsdraht 9 gerade so knapp, dass der Führungsdraht sich noch gut bewegen kann gegenüber dem Innenkatheter. Damit entstehen insgesamt kleine Abmessungen für den Katheter 2. Das ist erwünscht, damit während des Einsatzes der Gefässstütze 5 das im Gefäss 1 strömende Medium möglichst wenig durch den Katheter 2 behindert wird.

Der Draht 9 kann auch ein Führungsdraht sein, der nicht nur an der Punktionsstelle benutzt wird, sondern der generell zum Einsatz des Katheters 2 im Körper vorgeschoben wird und dem der Katheter 2 dann folgen kann. Es kann aber auch ein Führungsdraht sein, der von einer vorhergehenden Behandlung schon bis zur Behandlungsstelle im Körper vorgeschoben ist und dort noch liegt, beispielsweise von der Behandlung mit einem Ballonkatheter.

In dem Gefäss 1 ist auch eine Gefässstütze 5 dargestellt. Die Gefässstütze 5 ist aus einem durchlässigen Netz sich überkreuzender steifer Fäden 6 angefertigt. Die Steifigkeit der Fäden 6 und das Verfahren zur Herstellung des Netzes ist so ausgewählt, dass die Gefässstütze 5 bei ihrem Einsatz sich aus einem gespannten Zustand mit kleinem Umfang durch ihre radiale Elastizität aus eigener Kraft aufweitet in einen die Gefässwand stützenden entspannten Zustand mit über die Länge gleichbleibendem grossem Umfang.

Die steifen Fäden 6, die das Netz bilden, sind schraubenförmig gelegt und überkreuzen sich. Sie haben damit einen bestimmten Winkel zur Längsachse des Gefässes und können z. B. durch ihre Spiralform sich an den jeweils gegenüber liegenden Gefasswänden abstützen. Dadurch wird eine relativ hohe aber vor allem auch eine über die Länge des Fadens und über die Länge der Gefässstütze gleichbleibende Anpresskraft erreicht. Fäden ohne Schraubenform können sich nur an ihren jeweiligen Wurzel- und Endpunkten abstützen, in der Mitte zwischen diesen Abstützungen lässt ihr gegen die Gefässwand gerichteter Stützdruck nach, sobald sie nicht mehr punktförmig sondern über eine bestimmte Gefässlänge an der Gefässwand anliegen.

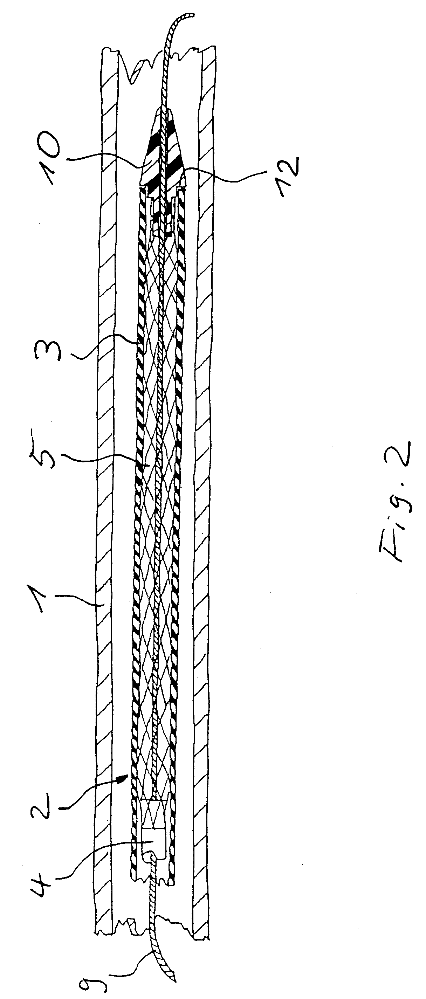

In Fig. 2 ist die Gefässstütze 5 in gespanntem Zustand zu sehen. Die Gefässstütze 5 liegt eingespannt auf einen kleinen Umfang zusammengezogen am distalen Ende des schlauchförmigen äusseren Katheterschafts 3 gefangen. Um die Gefässstütze 5 zu öffnen und zu ihrem Einsatz in ihren entspannten Zustand freizugeben in dem sie selbstexpandierend sich aufweitet, sich an die Gefässwand anlegt und diese stützt, wird der äussere Katheterschaft 3 gegenüber dem Innenkatheter 4 zurückgezogen.

Wenn der Aussenkatheter 3 gegenüber dem Innenkatheter zurückgezogen wird, muss der Innenkatheter 4 die im äusseren Katheterschaft 3 an der Innenwand anliegende Gefässstütze 5 an ihrem proximalen Ende axial abstützen, damit eine Relativbewegung zwischen äusserem Katheterschaft 3 und Gefässstütze 5 zustande kommen kann.

Besonders in Fig. 1 ist zu sehen, dass die Gefässstütze an ihrem proximalen, bedienungsnahen Ende eingespannt so gesichert ist, dass sie einen durchlässigen Netzkegel 7 bildet. Der Netzkegel 7 ist aus denselben steifen Fäden 6 hergestellt, die auch die Gefässstütze 5 bilden, sodass sich der Kegel 7 zusammen mit der Gefässstütze selbst öffnet. Der Radius dieses Netzkegels 7 steigt stetig zum Radius der Gefässstütze 5 an. An seiner Spitze ist der Netzkegel 7 auf den Aussendurchmesser des Innenkatheters 4 zusammengezogen. Er ist dort durch eine Schicht Bindemittel mit dem Innenkatheter verbunden. Mit Hilfe des Netzkegels 7 ist daher die Gefässstütze 5 an der Verbindungsstelle 8 fest am Innenkatheter 4 verankert.

Der Innenkatheter 4 hat zum Öffnen und Schliessen der Gefässstütze 5 lediglich Druck- und Zugkräfte zu übertragen. Er kann daher in seiner Wandstärke sehr dünn gehalten werden. Das führt ebenfalls zu geringen Aussenabmessungen des Katheters 2, die den Medienstrom im Gefäss wenig behindern.

Die anderen Enden der steifen Fäden 6 sind, nachdem sie die Gefässstütze 5 gebildet haben, wieder zusammengeführt und sind distal an einer Spitze 10 verankert. Die Fäden bilden dadurch an ihrem distalen Ende einen zweiten, zum ersten spiegelbildlichen Netzkegel 11.

Die Spitze 10, an der die Fäden 6 verankert sind, ist in Fig. 2 im Schnitt dargestellt. In ihrem Inneren lässt sie den Führungsdraht 9 durch. Der Führungsdraht 9 ist frei beweglich gegenüber der Spitze 10, sodass die Spitze 10 beim Öffnen und Schliessen der Gefässstütze ihre Lage zum distalen Ende des Innenkatheters 3 frei verändern kann und dass der Führungsdraht 9 unabhängig vom Katheter 2 bewegt werden kann. Die Passage für den Führungsdraht 9 durch die Spitze 10 zentriert gleichzeitig den Führungsdraht 9 gegenüber dem Katheter 2.

In der in Fig. 2 dargestellten Lage der Gefässstütze 5, in der der Aussenkatheter 3 die Gefässstütze 5 gespannt vollständig in sich aufnimmt, verschliesst die Spitze 10 die distale Öffnung des Aussenkatheters 3. Sie ist durch einen an der Spitze 10 angeformten Zapfen 15 im Aussenkatheter 3 zentriert. Die Spitze 10 kann zum Verschliessen der distalen Öffnung des Aussenkatheters 3 diesen z.B. ganz ausfüllen. In den gezeigten Ausführungsbeispielen weist sie eine Schulter 12 auf, die zum Abdichten den Aussenkatheter 3 übergreift. Beim Vorschieben des Aussenkatheters 3 schlägt dieser mit seinem distalen Rand an die Schulter 12 an. Der Aussenkatheter 3 wird dadurch zuverlässig abgedichtet und der Arzt hat einen spürbaren Anschlag, als Kontrolle, wann die Gefässstütze vollständig zurückgefaltet ist. An der Stelle der Schulter 12 ist der Durchmesser der Spitze im wesentlichen gleich dem Durchmesser des Aussenkatheters 3. Der Rand der distalen Aussenkatheteröffnung kann dadurch beim Einführen des Katheters 2 in das Gefäss 1 oder beim Passieren von Engstellen im Gefäss 1 nicht hängenbleiben.

Die Spitze 10 verjüngt sich zu ihrem distalen Ende und ist distal von der Schulter 12 wie eine Pfeilspitze kegelförmig ausgebildet mit der Passage für den Führungsdraht 9 in der Mitte angeordnet und an der Kegelspitze austretend. Wenn sie den Aussenkatheter 3 verschliesst, erstreckt die Spitze 10 sich soweit nach distal, dass beim Vorschieben des Katheters 2 Verletzungen der Gefässwand durch den Aussenkatheter 3 vermieden werden. Bei der in den Zeichnungen gezeigten Spitze 10 ist diese distale Ausdehnung der Spitze 10 so gross, dass der volle Aussendurchmesser des Katheters 2 nicht stumpfwinklig auf die Gefässwand 1 auftreffen kann. Auf diese Weise wird der Katheter 2 sicher entlang der Gefässlängsachse geleitet und er kann ohne Widerstände vorgeschoben werden. Dabei verhindert der Führungsdraht 9, auf dem die Spitze 10 reitet, eine Verletzung der Gefässwand durch die an der Spitze 10 vorgesehene Kegelspitze.

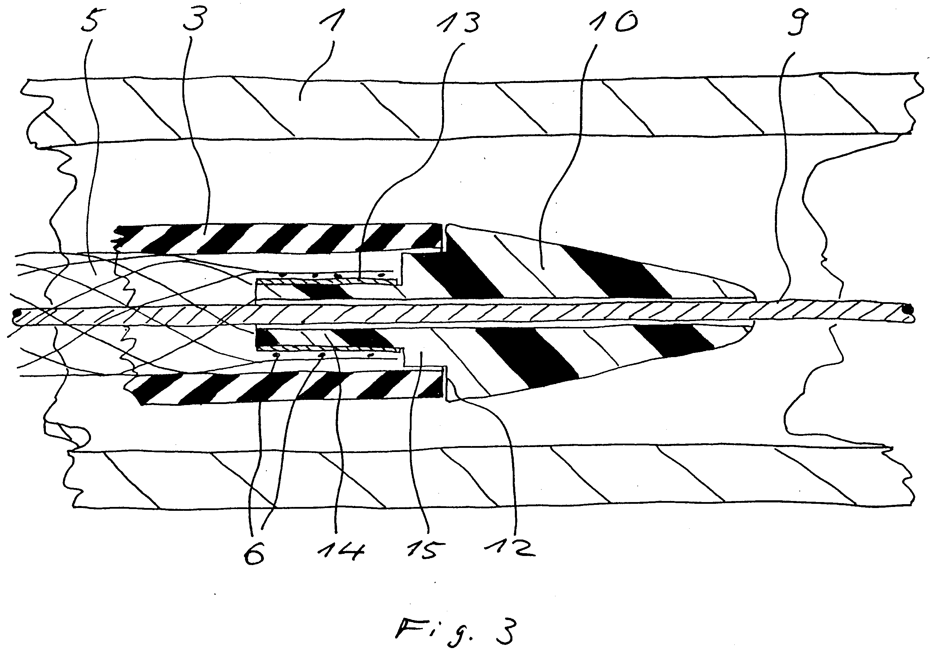

Die Spitze 10 kann z.B aus einem röntgenologisch erkennbaren Material hergestellt sein. In Fig. 3 ist ein Ausführungsbeispiel gezeigt, bei dem an der Spitze 10 ein röntgenopaker Ring 13 angebracht ist. Die Spitze 10 selbst muss dann nicht röntgenopak sein. Der Ring 13 ist auf einen am Zapfen 15 ausgebildeten Absatz 14 aufgeschoben. Dieser Absatz 14 dient auch zur Verankerung der Netzfäden 6 an der Spitze 10. Die Fäden 6 sind durch Bindemittel, durch Löten oder durch Schweissen mit dem Ring 13 verbunden.

Zum Gebrauch liegt die Gefässstütze 5 zunächst gespannt am distalen Ende innerhalb des äusseren Katheterschaftes 3 im Katheter 2. Der Innenkatheter 4 ist zurückgezogen gegenüber dem äusseren Katheterschaft 3 und die Gefässstütze 5 legt sich gegen die Innenwand des äusseren Katheterschaftes 3 wie in Fig. 2. Der Innenkatheter ist soweit zurückgezogen, dass auch das distale Ende der Gefässstütze 5 innerhalb des äusseren Katheterschaftes 3 liegt. Die Spitze 10 verschliesst jetzt die distale Öffnung des Aussenkatheters 3 und dient bei Bedarf als Dilator, als Aufweiter, um z.B. die Punktionsstelle oder eine Engstelle im Gefäss aufzuweiten.

In diesem Zustand wird der Katheter 2 in das Gefäss 1 eingeführt und in dem Gefäss 1 vorgeschoben. Wenn das distale Ende des Katheters 2 die Behandlungsstelle passiert hat, wird der äussere Katheterschaft 2 gegenüber dem stationär gehaltenen Innenkatheter 4 zurückgezogen. Durch die Verbindung der Gefässstütze 5 mit dem stationär gehaltenen Innenkatheter 4 kommt eine Relativbewegung zwischen Gefässstütze und äusserem Katheterschaft 3 zustande. Dadurch wird die Gefässstütze 5 langsam Stück für Stück, von ihrem distalen Ende her anfangend, freigegeben. Sie tritt aus dem distalen Ende des äusseren Katheterschaftes 3 aus und weitet sich langsam auf in ihren entspannten Zustand, in dem sie sich an die Gefässwand anlegt und sie stützt. Die Gefässstütze wird nun so weit wie erforderlich freigegeben, z.B. bis die Verbindungsstelle 8 aus dem distalen Ende des äusseren Katheterschaftes 3 ausgetreten ist wie in Fig. 1.

Soll nun die Gefässstütze 5 wieder aus dem Gefäss 1 entfernt werden, so muss der äussere Katheterschaft 3 gegenüber dem Innenkatheter 4 lediglich wieder vorgeschoben werden. Durch die Verbindung der Gefässstütze 5 mit dem Innenkatheter 4 ergibt sich auch daraus wieder eine Relativbewegung zwischen Gefässstütze und äusserem Katheterschaft 3. Durch die Verbindung der Gefässstütze 5 mit dem Innenkatheter 4 mit Hilfe des Netzkegels 7, dessen Spitze am Innenkatheter 4 befestigt ist, gleitet der Katheterschaft 3 aussen auf dem Netz der Gefässstütze 5 entlang und zwingt dadurch den vom Innenkatheter 4 aus ansteigenden Netzkegel 7 und die daran anschliessende Gefässstütze 5 wieder in ihre gespannte Form zurück in der sie wieder vom Katheterschaft 3 aufgenommen werden kann. Sobald die Gefässstütze sich von der Gefässwand gelöst hat, kann auch der der Innenkatheter 4 gegenüber dem äusseren Katheterschaft 3 zurückgezogen werden. Die Gefässstütze 5 kann auf diese Weise wieder ganz in den äusseren Katheterschaft 5 zurückgezogen werden und der Katheter 2 kann aus dem Gefäss 1 entfernt werden oder der Katheter 2 kann verschoben werden und die Gefässstütze 5 kann an einer korrigierten Stelle erneut ausgebracht werden.

Ein vorteilhaftes Verfahren, einen Katheter 2 nach der Erfindung herzustellen besteht darin, die konvergierenden Enden der beiden Netzkegel 7,11 der Gefässstütze 5 jeweils durch einen Schrumpfschlauch auf dem Aussendurchmesser des entsprechenden Verankerungsbettes am Innenkatheter 3 bzw. auf der Spitze 10 zusammenzuhalten, während die Netzkegel 7,11 mit dem Innenkatheter 3 bzw. mit der Spitze 10 verbunden werden. Der Schrumpfschlauch wird in seinem Rohzustand in dem er noch einen grossem Durchmesser hat, über die Gefässstütze 5 gezogen. Der Schrumpfschlauch wird dann erwärmt, er zieht sich dadurch zusammen und schnürt damit auch die Gefässstütze 5 ein, die er umgibt. Abmessungen und Material des Schrumpfschlauches sowie die Wärmezufuhr zum Schrumpfschlauch können so ausgewählt werden, dass der Schrumpfschlauch die Gefässstütze 5 auf den Aussendurchmesser des Innenkatheters 4 bzw. den Aussendurchmesser des Absatzes 14 zusammenzieht und dort zusammenhält. In diesem Zustand kann dann die Gefässstütze 5 mit dem Innenkatheter 4 verschweisst werden oder eine vorher angebrachte oder durch Kapillarwirkung unter den Schrumpfschlauch in die Verbindungsstelle 8 hinein wandernde Schicht Bindemittel kann aushärten. Nach diesem Vorgang kann der Schrumpfschlauch wieder entfernt werden, sodass, wie in den Fig. 1 und 2 dargestellt, eine glatte Verbindungsstelle 8 übrig bleibt, die, je nach gewähltem Verfahren, im wesentlichen den Durchmesser des Innenkatheters 4 aufweisen kann.

## Patentansprüche

1. Katheter mit einer zylindrischen Gefässstütze (5) aus einem durchlässigen Netz sich überkreuzender steifer Fäden (6), wobei die Gefässstütze (5) bei ihrem Einsatz sich aus einem gespannten Zustand mit kleinem Umfang durch ihre radiale Elastizität aus eigener Kraft aufweitet in einen die Gefässwand (1) stützenden entspannten Zustand mit über die Länge gleichbleibendem grossem Umfang, mit einem schlauchförmigen äusseren Katheterschaft (3), der an seinem distalen Ende die gespannte Gefässstütze (5) in sich aufnimmt und aus dem die Gefässstütze (5) zu ihrem Einsatz freigegeben werden kann, mit einem Innenkatheter (4) im Innern des schlauchförmigen äusseren Katheterschafts (3), der im Aussenkatheter (3) verschieblich ist, der für eine direkte Übertragung der Verschiebewege entlang seiner Länge im Aussenkatheter (3) gelagert ist und der in seinem Inneren eine Passage für einen Führungsdraht (9) freilässt, die er eng umschliesst, wobei zur Freigabe der Gefässstütze (5) der äussere Katheterschaft (3) gegenüber dem Innenkatheter (4) zurückgezogen wird und wobei die Gefässstütze (5) an ihrem proximalen Ende im eingespannten Zustand so gesichert ist, dass sie einen mit der Gefässstütze (5) sich selbst öffnenden durchlässigen Netzkegel (7) bildet, dessen Radius stetig zum Radius der entspannten Gefässstütze (5) ansteigt und wobei die Gefässstütze (5) mit Hilfe des Netzkegels (7) fest an dem lediglich Druck- und Zugkräfte übertragenden Innenkatheter (4) verankert ist, dadurch gekennzeichnet, dass die das Netz bildenden steifen Fäden (6) an ihren Enden wieder zusammengeführt werden und distal an einer Spitze (10) verankert sind und dadurch am distalen Ende der Gefässstütze (5) einen zweiten, zum ersten spiegelbildlich liegenden Netzkegel (11) bilden, wobei die Spitze (10), an der die Fäden (6) verankert sind, in ihrem Inneren den Führungsdraht (9) durchlässt und nach aussen so geformt ist, dass sie die distale Öffnung des Aussenkatheters (3) verschliesst wenn der Aussenkatheter (3) die Gefässstütze (5) gespannt vollständig in sich aufnimmt, wobei die Spitze (10), wenn sie den Aussenkatheter (3) verschliesst, mit ihrem distalen Ende gegenüber der distalen Öffnung des Aussenkatheters (3) sich verjüngend soweit nach distal erstreckt, dass der volle Aussendurchmesser des Katheters (2) nicht stumpfwinklig auf die Gefässwand (1) auftreffen kann.

2. Katheter nach Anspruch 1, dadurch gekennzeichnet, dass die Spitze (10) eine Schulter (12) aufweist, die den Aussenkatheter (3) übergreift.

3. Katheter nach Anspruch 1 oder 2, dadurch gekennzeichnet, dass die Spitze (10) röntgenologisch erkennbar ist.

4. Katheter nach Anspruch 3 dadurch gekennzeichnet, dass an der Spitze (10) ein radiopaker Ring (13) angebracht ist, auf dem die distalen Enden der Gefässstützenfäden (6) verankert sind.

5. Verfahren zur Herstellung eines Katheters nach einem der Ansprüche 1 bis 4, dadurch gekennzeichnet, dass die konvergierenden Enden der beiden Netzkegel (7, 11) der Gefässstütze (5) jeweils durch einen Schrumpfschlauch auf dem Aussendurchmesser (3) des entsprechenden Verankerungsbettes am Innenkatheter (4) bzw. auf der Spitze (10) zusammengehalten werden während die Netzkegel (7, 11) mit dem Innenkatheter (4) bzw. mit der Spitze (10) verbunden werden.

## Claims

1. Catheter with a cylindrical vessel support (5) consisting of a permeable mesh of intersecting stiff threads (6), wherein the vessel support (5) expands in use owing to its radial elasticity due to is own force from a tensioned state with a small circumference into a relaxed state supporting the vessel wall (1) with a large circumference which is uniform over the length, with a tubular outer catheter shank (3) which receives the tensioned vessel support (5) at its distal end and from which the vessel support (5) can be released for use, with an internal catheter (4) in the interior of the tubular outer catheter shank (3) which is displaceable in the external catheter (3), is mounted in the external catheter (3) for direct transfer of the displacements along its length and leaves in its interior a passage for a guide wire (9) which it surrounds closely, wherein the outer catheter shank (3) is retracted relative to the internal catheter (4) to release the vessel support (5) and wherein the vessel support (5) is secured at its proximal end in the fixed state so as to form a permeable mesh cone (7) which opens with the vessel support (5) itself and of which the radius increases continuously to the radius of the relaxed vessel support (5) and wherein the vessel support (5) is secured firmly on the internal catheter (4) which merely transmits compressive and tensile forces, by means of the mesh cone (7), characterised in that the stiff threads (6) forming the mesh are brought together again at their ends and are secured distally on a tip (10) and thus form a second mesh cone (11) in a mirror image to the first one at the distal end of the vessel support (5), wherein the tip (10) on which the threads (6) are secured allows the passage of the guide wire (9) in its interior and is shaped externally such that it closes the distal orifice of the external catheter (3) when the external catheter (3) completely receives the relaxed vessel support (5), wherein the tip (10), when it closes the external catheter (3), extends with its distal end remote from the distal orifice of the external catheter (3) while tapering to such an extent distally that the total external diameter of the catheter (2) cannot meet the vessel wall (1) bluntly.

2. Catheter according to claim 1, characterised in that the tip (10) has a shoulder (12) which overlaps the external catheter (3).

3. Catheter according to claim 1 or 2, characterised in that the tip (10) can be detected radiologically.

4. Catheter according to claim 3, characterised in that a ring (13) which is opaque to X-rays and on which the distal ends of the vessel support threads (6) are secured is arranged on the tip (10).

5. Method of producing a catheter according to one of claims 1 to 4, characterised in that the convergent ends of the two mesh cones (7, 11) of the vessel support (5) are held together in each case by a shrink tube on the external diameter (3) of the corresponding securing bed on the internal catheter (4) or on the tip (10) while the mesh cones (7, 11) are connected to the internal catheter (4) or to the tip (10).

## Revendications

1. Cathéter avec un support vasculaire cylindrique (5) réalisé sous forme d'un réseau de fils rigides (6) croisés, le support vasculaire (5) s'élargissant par élasticité radiale, lors de son utilisation, par sa propre force d'un état tendu à petite circonférence vers un état détendu à grande circonférence, constante sur toute la longueur dans lequel il soutient la paroi vasculaire (1), le cathéter comportant une tige extérieure (3) en forme de tuyau flexible dont l'extrémité distale accueille le support vasculaire tendu (5) et de laquelle le support vasculaire (5) peut être libéré pour être utilisé avec un cathéter intérieur (4) disposé à l'intérieur de la tige de cathéter extérieur (3) en forme de tuyau flexible, qui peut coulisser dans le cathéter extérieur (3), qui est disposé dans le cathéter extérieur (3) pour la transmission directe des trajets de coulissement selon sa longueur, et qui ménage dans son intérieur un passage pour un fil de guidage (9) qu'il entoure étroitement, la tige de cathéter extérieur étant rétractée par rapport au cathéter intérieur (4) pour libérer le support vasculaire (5), l'extrémité proximale du support vasculaire (5) étant maintenue, à l'état monté, de manière à former un cône réticulé (7) perméable qui s'ouvre automatiquement avec le support vasculaire (5) et dont le rayon augmente progressivement vers le rayon du support vasculaire détendu (5), le support vasculaire (5) étant fermement ancré, à l'aide du cône réticulé (7), sur le cathéter intérieur (4) qui ne transmet que des forces de pression et de traction,
caractérisé en ce que les extrémités des fils rigides (6) qui forment le réseau sont réunis de nouveau et sont ancrés distalement à une pointe (10) formant ainsi à l'extrémité distale du support vasculaire (5) un deuxième cône réticulé (11) disposé inversement au premier, la pointe (10) à laquelle les fils (6) sont ancrés laissant passer à son intérieur le fil de guidage (9) et étant formée vers l'extérieur de manière à obturer l'ouverture distale du cathéter extérieur (3) lorsque le cathéter extérieur (3) accueille totalement le support vasculaire tendu (5), la pointe (10) s'étendant en s'effilant, lorsqu'elle obture le cathéter extérieur (3), en regard de l'ouverture distale du cathéter extérieur (3) suffisamment dans la direction distale pour que le diamètre extérieur complet du cathéter (2) ne puisse pas rencontrer la paroi vasculaire (1) sous un angle obtus.

2. Cathéter selon la revendication 1,
caractérisé en ce que la pointe (10) comporte une épaule (12) qui chevauche le cathéter extérieur (3).

3. Cathéter selon la revendication 1 ou 2,
caractérisé en ce que la pointe (10) peut être identifiée radiologiquement.

4. Cathéter selon la revendication 3,
caractérisé en ce qu'une bague radiopaque (13) est disposée sur la pointe (10), les extrémités distales des fils (6) du support vasculaire étant ancrées sur cette bague.

5. Procédé de réalisation d'un cathéter selon l'une des revendications 1 à 4,
caractérisé en ce que les extrémités convergentes des deux cônes réticulés (7, 11) du support vasculaire (5) sont respectivement tenues rassemblées par un tuyau contractible, sur le diamètre extérieur (3) du lit de fixation respectif du cathéter intérieur (4) ou de la pointe (10) pendant que les cônes réticulés (7, 11) sont reliés au cathéter intérieur (4) ou à la pointe (10).
